# NEW EUROPEAN PATENT SPECIFICATION

(11) **EP 0 554 291 B2**
(45) Date of publication and mention of the opposition decision: **20.10.2010**
(45) Mention of the grant of the patent: 17.12.2003
(21) Application number: 91918144.6
(22) Date of filing: 17.10.1991
(51) Int. Cl.: A61K 31/60, A61K 31/606, A61K 31/635, A61P 1/00

(54) **TREATMENT OF NON-INFLAMMATORY BOWEL DISORDERS**
BEHANDLUNG VON NICHT-ENTZÜNDLICHEN DARMERKRANKUNGEN
TRAITEMENT D'AFFECTIONS INTESTINALES NON-INFLAMMATOIRES

(30) Priority: 22.10.1990 AU 295090
(43) Date of publication of application: 11.08.1993
(73) Proprietor: CENTRE FOR DIGESTIVE DISEASES PTY LTD, Five Dock, NSW 2046 (AU)
(72) Inventor: Borody, Thomas Julius, New South Wales 2046 (AU)
(74) Representative: Evans, Claire
(86) International application number: PCT/AU1991/000482
(87) International publication number: WO 1992/006690

(56) References cited:
- WO-A1-81/02671
- WO-A1-81/02672
- WO-A1-92//06690
- AU-A- 5 367 290
- GB-A- 2 021 409
- US-A- 4 664 256
- DISEASES OF THE COLON AND RECTUM vol. 27, no. 8, 1984, pages 513 - 514 SLADEN, G.E. ET AL 'IS SEGMENTAL COLITIS A COMPLICATION OF DIVERTICULAR DISEASE?'
- GASTROENTEROLOGY vol. 94, no. 5, May 1988, page A426 SHURBERG, J.L. 'A 10 YEAR REVIEW OF CHRONIC DIARRHOEA AND IRRITABLE BOWEL SYNDROME (IBS)'
- GASTROENTEROLOGY vol. 102, no. 4, April 1992, page A428 BORODY, T.J. ET AL 'USE OF 5-AMINOSALICYLIC ACID IN IRRITABLE BOWEL SYNDROME'
- Biochemical Pharmacology, Volume 35, No. 2 issued 1986 (Great Britain) ROEDIGER W et al, "Effect of 5-amino salicyclic acid (5-ASA) and other salicylates on Short Chain Fat Metabolism in Colonic Mucosa", see pages 221-225.
- Biochemical Pharmacology, Vol 38, No 1, issued 1989 (Great Britain), WILLIAMS J G and HALLETT, M B 'The Reaction of 5-Amino-Salicylic Acid Hypochlorite' see pages 149-154.
- Mims Annual 1989, 13th Edition by IMS Publishing (Australia) see pages 1-23.
- The Merck Index 1989, 11th Edition by Merck & Co Inc (USA) see page 1411 Drug No.8917.
- H. SIEBNER, M. BRYDE THER. D. GEGENW. vol. 114, 1975, pages 982 - 992

## Description

### TECHNICAL FIELD

This invention relates to the production and use of therapeutic agents for the treatment of certain bowel disorders, namely disorders arising from unknown or non-obvious causes, which are unaccompanied by inflammation and are not due to detectable infection by known pathogenic organisms. Such disorders are referred to as non-specific bowel disorders hereinafter, and may be distinguished from specific bowel disorders having a diagnosable cause which may be treated by appropriate medication or surgery. Typical non-specific bowel disorders are irritable bowel syndrome (IBS), chronic constipation, non-ulcer dyspepsia (NUD), gastro-oesophageal reflux with or without oesophagitis (GOR), and diverticular disease.

### BACKGROUND ART

The human large bowel (colon), and to a lesser extent the small bowel, contain large concentrations of various enteric bacteria. They may range in concentration from between 10² to 10⁷ per cubic centimetre in the small bowel and up to 10¹⁴ per cubic centimetre in the large bowel. When the bacteria are non-pathogenic, then the bowel produces no symptoms in the body.

On the other hand when the normal bowel flora is invaded or joined by pathogenic bacterial strains which may colonise the bowel and remain there long-term, chronic illness can result.

The local effects of abnormal bowel flora may include abdominal cramps caused by colonic or small bowel contraction, distension caused by either fluid or gas accumulation, diarrhoea caused by inadequate fluid absorption or excessive secretion, or constipation by abnormal motility patterns and excessive absorption of water. Severe local effects of abnormal bowel flora can include microscopic or collagenous colitis, ulcerative colitis, Crohn's disease and diverticulosis. Some of these effects are caused by local toxins, others by invasion of bacteria into the bowel lining and in others, the mechanisms are unknown.

When obvious, visible or microscopic colitis is present, it is known that beneficial clinical effects can be obtained from well known anti-bacterial drugs derived from salicylic acid, such as sulfasalazine (prepared by coupling 2-sulfanilamidopyridine with salicylic acid), 4-aminosalicylic acid, and 5-aminosalicylic acid.

When there are no visible abnormalities detectable in the colon and when stool tests, histology and blood tests are negative, yet patients still complain of symptoms referrable to the colon, a diagnosis of IBS will often be made. Some 10-25% of the Western population suffer with this disorder which has also been termed spastic or irritable colon, unstable colon, colonic neurosis, spastic colitis or mucus colitis. In the classic case, there is a triad of symptoms including lower abdominal pain relieved by defecation, alternating constipation and diarrhoea and the passage of small calibre stools. Abdominal distension, flatulence or wind are also frequently present, as is passage of mucus as well as the sensation of incomplete evacuation. All these symptoms are present in the absence of demonstrable organic disease.

The pathogenesis of IBS has hitherto been unknown. Emotional disturbance, fibre deficiency, purgative abuse and food intolerance have all been implicated but not proven nor well demonstrated. Evidence for infection or autoimmunity is lacking. Conventional treatments for IBS have been unsatisfactory, as instanced by the very number of therapies that have, from time to time, been recommended or trialled. These have ranged from psychotherapy and dietary regimes to medication by antispasmodic agents, anticholinergic agents, barbiturates, antidepressants, bulking agents, dopamine antagonists, carminatives, opioids, and tranquillisers; all without signal success. There is no evidence that cure is possible.

IBS is one of the most common of the gastrointestinal illnesses, and though not life-threatening, causes great distress to those severely afflicted and brings a feeling of frustration and helplessness to the physicians attempting to treat it.

### DISCLOSURE OF THE INVENTION

The present invention arose from observations by the applicant that treatments of patients for other complaints requiring the administration of antibiotics appeared sometimes to produce beneficial results in respect of IBS and other non-specific bowel disorders. This led to the hypothesis that as yet unproven and undocumented bowel flora alterations or infection by mildly pathogenic bacteria constitutes the mechanism which underlines the pathogenesis of non-specific bowel disorders. Having postulated infection as being the cause of irritable bowel syndrome and the other above-mentioned enteric afflictions, the applicant conducted clinical trials which have shown that 5-aminosalicylic acid compounds are capable of suppressing the symptoms in most patients provided the appropriate dose is administered.
The invention consists in the use of 5-aminosalicylic acid (5-ASA) in the manufacture of a medicine for use in the treatment of non-inflammatory bowel disorders.

5-ASA may be used in relation to constipation, NUD, GOR diverticular disease, or IBS.

In each case the 5-ASA may be used in a manner similar to its use for the treatment of inflammatory bowel disease.

Thus the active ingredient may be incorporated with a pharmaceutically acceptable excipient in tablets or capsules. The capsules or tablets may be taken once at night, twice daily or three or more times daily, in dosages ranging from 200mg through to 18 grams per day. 5-ASA is usually administered in tablet form in a dosage of from 250mg per day to 18 grams per day in divided doses. All the 5-amino salicylic acid agents have to be prepared in such a way that they are released in the distal small bowel. This requires the agent to be furnished with an enteric coating or provided in an enteric coated release capsule. If 5-ASA is released in the upper small bowel and is absorbed to any extent, then it is secreted in the kidney and cuases kidney damage because of crystal formation. Suitably coated or encapsulated products are already available for other purposes, for example those marketed under the names olsalazine, salazopyrin or Mesasal.

As a general rule for long term therapy the dosage will commence at a low level and build up to the desired full amount over a few weeks, and the invention extends to multiple packages of individual dosage units to be taken in sequence to provide such a gradual build up.

From the foregoing it will be appreciated that a completely new use has been discovered for 5-ASA in an area where previously there has been no known effective treatment.

### BRIEF DESCRIPTION OF THE DRAWINGS

Figure 1 is a tabulation of the symptoms and their severity of a group of patients as at the commencement of a clinical trial of the invention.
Figures 2, 3 and 4 are graphical representations of selected results of the said clinical trial.

### BEST MODE OF CARRYING OUT THE INVENTION

The best mode of carrying out the invention known to the applicant may be readily appreciated from the following description of two experimental case studies and subsequent clinical trials prompted by the apparent success of those cases.

### INDIVIDUAL CASE STUDIES

### Example 1.

A 31 year old nursing sister (HB) was investigated for chronic abdominal pain and frequent (2-6/day) loose motions with occasional constipation. Stool cultures, large and small biopsy, small bowel enema X-ray, full blood count and multiple biochemical tests revealed no abnormalities. In spite of use of added fibre, food exclusion diets and antispasmodics, the symptoms essentially continued. Introduction of sulfasalazine (Salazopyrin-EN) in a dose of 1 g. b.d. resulted in abolition of pain and reversal of loose motions to formed stools. Withdrawal of therapy brought on recrudescence of original symptoms. Recommencement of sulfasalazine again brought prompt relief. The patient continued to obtain the relief at 5 months follow-up. She was then changed to 5-ASA therapy (utilising the medication marketed as Dipentum) and has been able to continue with the same relief suppression for over a year and a half.

### Example 2

A 42 year old female sales representative (LA) presented with lower, and occasionally generalised, abdominal cramping together with predominantly loose motions. In spite of extensive gastrointestinal investigations no organic cause was found. Commencement of dosing with sulfasalazine 1g. b.d. was accompanied by relief of almost all symptoms within 4-5 days. At 3 weeks, however, the patient developed a pronounced rash and treatment was withdrawn. 5-ASA (Dipentum) was not immediately available. Symptoms recurred. When 5-ASA was commenced at a dose of 2 tablets twice daily, the pain and loose motions again abated.

5-ASA has also been used successfully to treat non-ulcer dyspepsia in a number of patients. The dosage commenced at 250mg per day and is increased to 1g a day in the usual situation. At weeks 3-6 the effects commence with patients noticing reduction in reflux symptoms and upper GI tract bloating, eructation and burning. Similarly, chronic constipation is controlled by sulfasalazine in doses mentioned above and by the aminosalicylic acid compounds in the majority of patients in the doses described.

### CLINICAL TRIAL

### Patients and Method

The study was carried out on patients referred by general practitioners to a Medical Centre for Digestive Diseases, for colonoscopic evaluation of abdominal discomfort and bowel disturbances severe enough to warrant further investigation. All patients gave informed consent to take part in the trial, which was conducted in accordance with the Revised Declaration of Helsinki.

Patients with the clinical diagnosis of IBS were offered entry into the trial if their symptom complex satisfied the Manning criteria and complied with the exclusion criteria.

Exclusion criteria were:
i colonoscopic abnormalities eg. visible colitis, polyps, carcinoma or diverticulosis;
ii histological abnormalities eg. collagenous or microscopic colitis;
iii coagulopathy;
iv pregnancy or lactation;
v significant clinical or laboratory evidence of pulmonary, hepatic or renal disease or dysfunction;
vi sensitivity to salicylates; and
vii need for non-steroidal anti-inflammatory drugs, steroids, anti-coagulants or antispasmodic agents.

The study was an open-label, single-institution, unblinded prospective pilot trial aimed at establishing whether a controlled double-blind trial is warranted.

### Medication

A 5-ASA formulation, namely olsalazine, (DIPENTUM; KABI-PHARMACIA) was used in the form of 250mg capsules. To reduce side effects the olsalazine was administered in a stepwise manner from 250mg b.d. in week 1, 500mg b.d. in week 2, to 750mg b.d. by week 3 and onwards. The dosage was maintained at 750mg b.d. for 6 weeks. Those patients unable to tolerate the 750mg b.d. dose were maintained on the highest dose tolerated.

### Symptom assessment

The severity of symptoms was assessed by the use of a visual analog scale at entry into the trial, at 5 weeks, at termination of trial (8 weeks) and at a 12 week follow-up consultation. The analog scale consisted of a line marked by numbers at equal intervals from 0 to 10. Zero indicated absence of symptoms while 10 represented symptoms severe enough to interfere with work or requiring medication. Assessed symptoms included abdominal pain/discomfort, constipation, diarrhoea, abdominal distension and flatulence. Symptom scores were tabulated and statistical analysis carried out using Students t-test.

### Results

Of 26 patients enrolled in the study, data from 23 who completed the entire trial was available for evaluation. One patient terminated the trial prematurely due to excessive headaches, while 2 failed to return at appropriate intervals for symptom follow-up. Included were 8 males and 15 females ranging in age from 24 to 74 years (average = 44.3 years). Estimated duration of IBS symptoms ranged from 3 to 35 years, the average being 10.2 years.

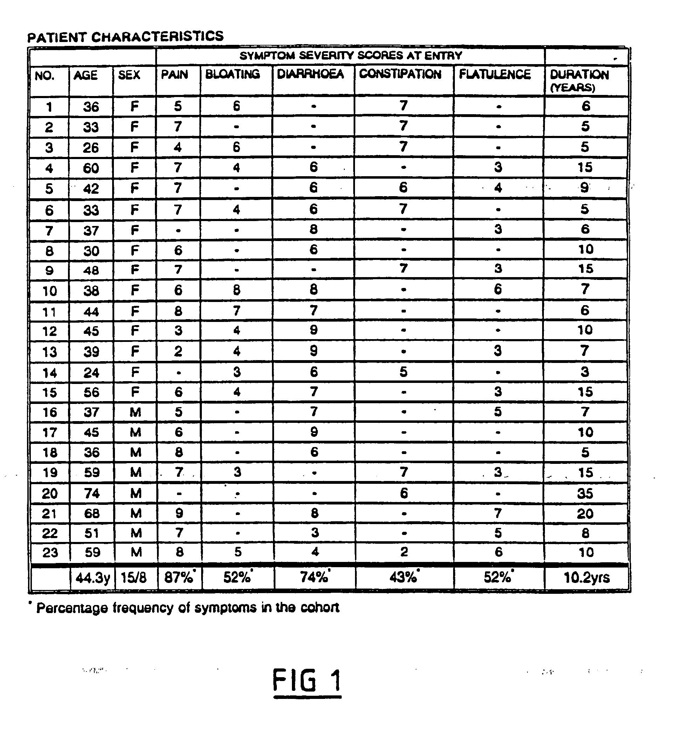

Patients' initial characteristics are summarised in Figure 1. Frequency and severity of the five evaluated symptoms at entry into the trial can be obtained from the listed visual analog scores. Most patients appeared to single out a dominant symptom by assigning higher scores.

### Symptom improvement

In a global assessment of their symptomatology most patients noted improvements in their dominant symptoms. Four patients of the 23 reported no appreciable change in their chief symptoms of pain (2) and constipation (2). The other nineteen patients reported a mean global improvement of 62% at 8 weeks. Abdominal pain, in particular, was significantly reduced. This was so whether the pain was diffuse, lower abdominal, left-iliac-fossa or left or right-upper-quadrant in location. Both diarrhoea and constipation also significantly improved towards normality. Flatulence and bloating symptoms showed lesser, sporadic improvement.
a. ABDOMINAL PAIN: Maximal improvement in pain scores occurred at 8 weeks. The initial score of 6.25± 1.74(SD) fell to 2.55± 1.47 (p<0.005). However, by week 12 the score returned to 5.7 ± 1.69, being 91% of the pain score at entry.
b. DIARRHOEA: Significant reduction in the diarrhoea score was noted at both the 5 and 8 week consultation. The change in symptom score fell from 6.77 ± 1.69 (SD) at entry to 2.35 ± 1.08 at 5 weeks and to 2.11 ± 0.60 at 8 weeks (p<0.005). The score rose to 4.18 ± 1.33 by 12 weeks.
c. CONSTIPATION: Significant improvement was also noted in the symptom scores for constipation. Baseline severity score reached its nadir at 8 weeks falling from 6.1 ± 1.60 (SD) to 2.3 ± 0.48 (P<0.005). The score rose again to pre-therapy values - 5.9 ± 1.99 - by the 12th week.
d. BLOATING/FLATULENCE: No significant reduction was found for these two symptoms on an overall assessment. In individual cases, however, occasional marked improvement occurred.

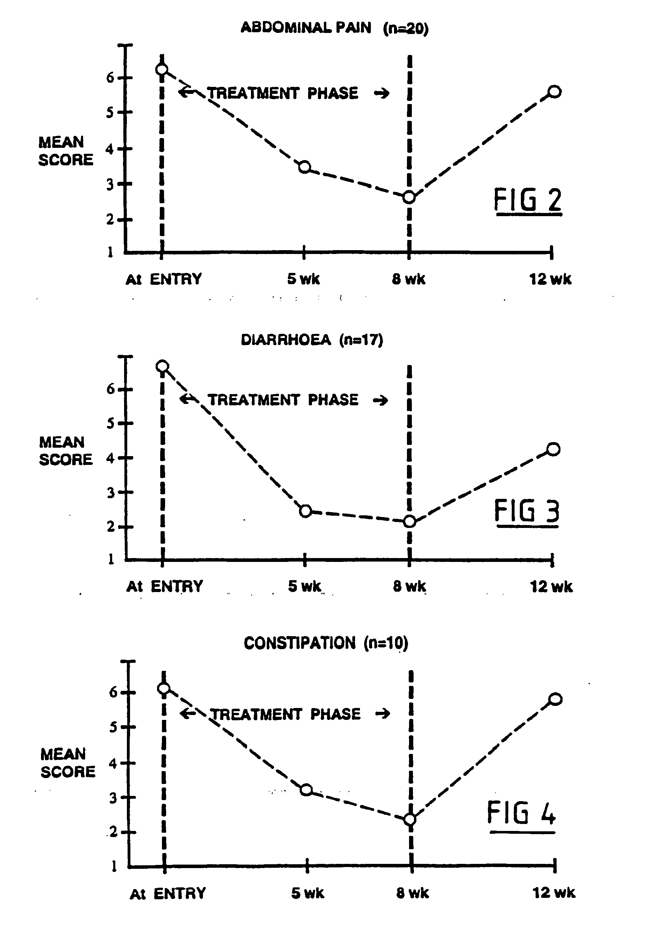

The above described results are shown in graphical form in Figures 2, 3 and 4, which represent the symptoms of abdominal pain, diarrhoea and constipation respectively, as assessed at intervals before, during and after treatment with 5-aminosalisylic acid (olsalazine). Not all patients experienced all three symptoms, the number of patients experiencing each symptom being indicated by the number n in each figure.

When asked if they would choose to take the medication on a long term basis 61% of patients expressed the desire to do so. Reasons for not wanting to continue medication included excessive intake of medication (6 capsules per day) or inadequate relief of symptoms for the therapy taken. Several patients specifically indicated that of the numerous therapies tried previously, olsalazine was the first to really improve their symptoms.

Six patients have continued to take olsalazine for between 8 and 21 months for the symptoms of pain, constipation or diarrhoea. Symptom control continued with maintenance therapy. Withdrawal of treatment resulted in recrudescence of symptoms which could again be suppressed by recommencement of olsalazine.

### Adverse Effects

Headache and nausea were recorded as the only discernible side effects of this slow, step-wise dose regimen. This occurred in four patients. No allergic reactions occurred. In one patient with constipation loose to normal motions were noted at a dose of 250 mg b.d., at which level the patient was therefore maintained.

## Claims

1. The use of 5-aminosalicylic acid in the manufacture of a medicine for use in the treatment of non-inflammatory bowel disorders.

2. The use of 5-aminosalicylic acid as claimed in claim 1, wherein the non-inflammatory bowel disorder is any one of constipation, non-ulcer dyspepsia, gastro-oesphageal reflux with or without oesophagitis and diverticular disease

3. The use of claim 1 wherein the medicine is manufactured as a dosage unit containing from 250mg to 1,000mg of 5-aminosalicylic acid.

4. The use of claim 1 wherein the medicine is manufactured as a multiple pack of dosage units comprising a first plurality of units each containing a predetermined dose of 5-aminosalicylic acid, a second plurality of units each containing a larger dose, and a third plurality of units each containing a still larger dose.

## Patentansprüche

1. Verwendung einer 5-Aminosalicylsäure, zur Herstellung eines Medikaments für die Verwendung bei der Behandlung von nichtentzündlichen Darmerkrankungen.

2. Verwendung einer 5-Aminosalicylsäure nach Anspruch 1, worin die nichtentzündliche Darmerkrankung Obstipation, Nicht-Ulkus-Dyspepsie, gastroösophagealer Reflux mit oder ohne Ösophagitis und durch Divertikel hervorgerufene Erkrankungen ist.

3. Verwendung nach Anspruch 1, worin das Medikament als Dosiseinheit mit 250 mg bis 1000 mg an 5-Aminosalicylsäure hergestellt wird.

4. Verwendung nach Anspruch 1, worin das Medikament als Mehrfachpackung von Dosiseinheiten, umfassend eine erste Vielzahl von Einheiten, die jeweils eine vorbestimmte Dosis der 5-Aminosalicylsäure enthalten, eine zweite Vielzahl von Einheiten, die jeweils eine höhere Dosis enthalten und eine dritte Vielzahl von Einheiten, die jeweils eine noch höhere Dosis enthalten, hergestellt wird.

## Revendications

1. Utilisation d'acide 5-aminosalicylique dans la fabrication d'un médicament destiné au traitement de désordres intestinaux non-inflammatoires.

2. Utilisation d'acide 5-aminosalicylique selon la revendication 1, selon laquelle le désordre intestinal non-inflammatoire est n'importe lequel de constipation, dyspepsie non ulcéreuse, reflux gastro-oesophagien avec ou sans oesophagite et diverticulose.

3. Utilisation selon la revendication, selon laquelle le médicament est fabriqué sous forme d'unité de dosage contenant 250 mg à 1,000 mg d'acide 5-aminosalicylique.

4. Utilisation selon la revendication 1, selon laquelle le médicament est fabriqué sous forme d'emballage multiple d'unités de dosage comprenant, une première pluralité d'unités contenant chacune une dose prédéterminée d'acide 5-aminosalicylique, une deuxième pluralité d'unités contenant chacune une plus grande quantité, et une troisième pluralité contenant chacune une quantité encore une plus grande.
